(19) **Europäisches Patentamt**
**European Patent Office**
**Office européen des brevets**

(11) **EP 3 703 629 B1**

(12) **EUROPÄISCHE PATENTSCHRIFT**

(45) Veröffentlichungstag und Bekanntmachung des Hinweises auf die Patenterteilung:
**07.05.2025 Patentblatt 2025/19**

(21) Anmeldenummer: **18796005.9**

(22) Anmeldetag: **30.10.2018**

(51) Internationale Patentklassifikation (IPC):
**A61F 9/008** (2006.01)

(52) Gemeinsame Patentklassifikation (CPC):
**A61F 9/00838;** A61F 2009/0087; A61F 2009/00895; A61F 2009/00897

(86) Internationale Anmeldenummer:
**PCT/EP2018/079677**

(87) Internationale Veröffentlichungsnummer:
**WO 2019/086436 (09.05.2019 Gazette 2019/19)**

(54) **VORRICHTUNG ZUR ERZEUGUNG EINER APERTURBLENDE IM AUGE**

DEVICE FOR CREATING AN APERTURE IN THE EYE

DISPOSITIF POUR PRODUIRE UNE OUVERTURE DANS L'OEIL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.10.2017 DE 102017125422**

(43) Veröffentlichungstag der Anmeldung:
**09.09.2020 Patentblatt 2020/37**

(73) Patentinhaber: **Rowiak GmbH**
**30419 Hannover (DE)**

(72) Erfinder: **LUBATSCHOWSKI, Holger**
**30989 Gehrden (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
EP-A1- 2 468 226     US-A1- 2004 199 149
US-A1- 2010 004 641

## Beschreibung

Die vorliegende Erfindung betrifft das Gebiet von Vorrichtungen, die zur Korrektur oder Minderung von Fehlsichtigkeiten im Auge vorgesehen sind, und insbesondere eine Vorrichtung zur Erzeugung einer Aperturblende in der natürlichen Linse des Auges, mit der die Schärfentiefe des Auges vergrößert wird, was insbesondere bei altersbedingter Weitsichtigkeit zu Verbesserungen führen kann.

US 2010/004641 A1 offenbart ein System und eine Vorrichtung zur Bestrahlung einer Linse eines Auges mit einem Laserstahl und stellt ein System und eine Vorrichtung zur Steigerung der Amplitude einer Akkommodation und/oder einer Änderung der Brechkraft und/oder zur Ermöglichung einer Entfernung von klarem oder getrübtem Linsenmaterial einer natürlichen kristallinen Linse bereit. Im Allgemeinen umfasst das System einen Laser, eine Optik zum Führen des Laserstrahls und ein Steuersystem zum Führen des Laserstrahls auf die Linse in einem vorbestimmten Muster. Ferner ist ein Bereichsbestimmungssystem zur Bestimmung der Form und Position der Linse hinsichtlich des Lasers vorgesehen. Zudem ist noch ein Verfahren und ein System zu Führen eines Laserstrahls in der Linse des Auges in einem vorbestimmten Schussmuster vorgesehen.

Eine Fehlsichtigkeit des Auges wird im Allgemeinen durch eine Brille oder durch Kontaktlinsen behoben. Im Falle der Ametropie wird ein optisch im Unendlichen liegender Gegenstand bei entspanntem Ziliarmuskel (Nah-Akkommodation) nicht scharf auf die Netzhaut abbildet. Ist der Augapfel im Vergleich zur Gesamtbrechkraft von Augenhornhaut und Augenlinse zu lang, spricht man von Myopie (Kurzsichtigkeit). Eine Brille oder Kontaktlinse, die als Zerstreuungslinse wirkt, kann die Brechkraft so verringern, dass ein scharfes Bild auf der Netzhaut abgebildet wird. Im umgekehrten Fall der Hyperopie (Weitsichtigkeit) ist die Brechkraft der Augenmedien zu gering im Verhältnis zur Augapfel-Länge. Hier kann eine Sammellinse als Brille oder Kontaktlinse die Fehlsichtigkeit korrigieren. Liegt die Fehlsichtigkeit nur in einer Ebene vor, spricht man vom Astigmatismus (Stabsichtigkeit). Entsprechende Zylinderlinsen können hier ebenfalls als Brille oder Kontaktlinse die Fehlsichtigkeit korrigieren.

Neben Brille oder Kontaktlinse als Korrekturhilfe gibt es chirurgische Verfahren, um die Fehlsichtigkeit des Auges zu korrigieren.

Zum einen lassen sich mit Lasern Teile der Augenhornhaut abtragen, womit die vordere Krümmung der Augenhornhaut so verändert wird, dass die sich optische Brechkraft des Auges entsprechend ändert und (annähernd) Normalsichtigkeit (Emmetropie) erreicht wird. Ein Vorteil derartiger Laserverfahren (PRK: photorefraktive Keratektomie, LASIK: Laser-in-situ-Keratomileusis, LASEK: Laser-epitheliale Keratomileusis) ergibt sich durch die Flexibilität der zu erreichenden Korrektur. Irreguläre Hornhautveränderungen lassen sich gezielt korrigieren, was mit Brille und Kontaktlinsen häufig nicht möglich ist.

Darüber hinaus lassen sich Implantate in die Hornhaut einbringen, die durch ihre optische Brechkraft oder durch ihre biomechanische Wirkung (etwa über eine Veränderung der Hornhautkrümmung) gezielt die Fehlsichtigkeit korrigieren sollen. Ebenso lassen sich Intraokularlinsen in das Auge implantieren, um Fehlsichtigkeiten zu korrigieren. Diese können zusätzlich zur natürlichen Augenlinse wirken oder die natürliche Augenlinse ersetzen.

Zu den Implantat-Lösungen gehört auch eine Gruppe von Implantaten, die das Prinzip der stenopäischen Lücke nutzbar machen (Lochblenden-Effekt). Die Lochblende reduziert störende Randstrahlen und vermindert dadurch sphärische Aberration bei der Bildgebung. Die Zerstreuungskreise auf der Netzhaut werden verkleinert und somit wird die Schärfentiefe bei der Bildgebung erhöht. Folglich erlangen ametrope Augen beim Blick durch eine Lochblende eine höhere Sehschärfe.

In einer Ausgestaltung eines solchen Ansatzes wird ein ca. 5 μm dickes Kunststoff-Scheibchen mit einem offenen Innendurchmesser von ca. 1,6 mm und einem Außendurchmesser von 3,8 mm zentral in die Hornhaut des Auges eingesetzt. Operativ geschieht dies meist dadurch, dass mit einem Laser eine Tasche in die Hornhaut präpariert wird, in der das Implantat fixiert wird.

In einer weiteren Ausgestaltung wird eine künstliche Intraokularlinse (IOL) in das Auge implantiert. Die natürliche Linse wird dabei entfernt. Innerhalb der künstlichen Linse befindet sich ebenfalls eine Lochblende mit ca. 1,36 mm freiem Innendurchmesser und 3,23 mm Außendurchmesser.

Schließlich kann der Lochblendeneffekt auch mit Hilfe einer Kontaktlinse nutzbar gemacht werden. Allerdings können sich Kontaktlinsen in der Regel auf der Augenhornhaut leicht bewegen. Die zentrale Öffnung wird dabei aus der Sehachse herausbewegt, so dass ein optimales Sehen beeinträchtigt wird.

Beispiele aus dem Stand der Technik, die sich auf eine Nutzbarmachung des Lochblendeneffektes beziehen, finden sich beispielsweise in US 4,955,904, US 5,757,458, US 5,980,040, WO 2011/020078 A1 und US 2013/131795 A1.

Brillen können im Alltag beschädigt werden oder verloren gehen. Je nach Umgebung können sie verschmutzen oder beschlagen. Unregelmäßige Fehlsichtigkeiten, jenseits von Myopie, Hyperopie oder Astigmatismus können nur unzulänglich oder gar nicht korrigiert werden, was auch für Kontaktlinsen gilt, die zudem eine gewisse Geschicklichkeit beim Einsetzen und Herausnehmen erfordern.

Chirurgische Eingriffe mit dem Laser und die Verwendung von Implantaten bergen die Gefahren einer möglichen Infektion während und nach der OP und / oder einer Unverträglichkeit des Gewebes mit dem Implantat. Von dem Einsatz des Lochblenden-Implantats in der Augenhornhaut ist bekannt, dass die Nährstoffversorgung des Gewebes beeinträchtigt werden kann (Alio, Jorge L.; et al. (2013): Removability of a small aperture intracorneal inlay for presbyopia correction. In: Journal of refractive surgery 29 (8), S. 550-556).

Ein der vorliegenden Erfindung zugrundeliegendes Ziel ist es insbesondere zu ermöglichen, die Lesefähigkeit presbyoper Augen wiederherzustellen. Es ist daneben oder darüber hinaus gewünscht, Sehfehler zu korrigieren, die durch Aberrationen, insbesondere in der Peripherie der optischen Achse des Auges entstehen, wobei die Randstrahlen des ins Auge einfallenden Lichtes ausgeblendet werden sollen. Hierbei sollen die Nachteile aus dem Stand der Technik ganz oder zumindest im Wesentlichen vermieden werden.

Es ist daher gewünscht, eine Lösung vorzustellen, bei der gewünschten Verbesserungen der Sehfähigkeiten möglichst ohne Einschränkungen im Alltag erreicht werden und die auch in der Ausführung der Behandlung selbst möglichst wenig Risiko mit sich bringt.

Erfindungsgemäß wird nach einem ersten Aspekt eine Vorrichtung zur Erzeugung einer Aperturblende in einem Auge vorgeschlagen, wie sie in Anspruch 1 definiert ist.

Ein weiterer Aspekt der Erfindung sieht ein Verfahren zur Erzeugung von Steuerbefehlen für eine Lasereinheit zur Erzeugung einer Aperturblende in einem Auge vor, wie es in Anspruch 9 definiert ist.

Da die Apertur (Lochblende) im Auge selbst geschaffen wird, treten die zum Fall eines äußeren Anbringens durch Brille oder Kontaktlinse diskutierten Probleme nicht auf. Da die Apertur auch nicht chirurgisch implantiert wird, besteht kein wesentliches Risiko einer Infektion oder einer Unverträglichkeit. Da Erfindung eine Anbringung der Apertur in nichtinvasiver Form ermöglicht, wird keine sterile Umgebung (Operations-Saal) benötigt. Es ist auch keine Stoffeinbringung (Farbstoffe, Pigmente) in das Auge vorgesehen, so dass auch in dieser Hinsicht keine Unverträglichkeiten zu befürchten sind.

Die vorliegende Erfindung bietet in Abkehr zum Stand der Technik den Vorteil, dass nicht invasiv, d.h. ohne das Auge chirurgisch zu eröffnen, eine Apertur in die Linse des Auges eingebracht werden kann. Die Apertur blendet Randstrahlen aus. Dadurch wird die Sehschärfe des Auges gesteigert. Insbesondere erhöht sich die Schärfentiefe und presbyope Augen verbessern dadurch die Nahsicht, beispielsweise die Lesefähigkeit.

Ein Teil des Hintergrunds der vorliegenden Erfindung findet sich in den folgenden Überlegungen.

Mit Hilfe insbesondere sogenannter "ultrakurzer" Laserpulse ist es möglich, im Inneren des Auges disruptive Prozesse zu erzeugen, ohne das Auge chirurgisch zu eröffnen. Der Wechselwirkungsmechanismus der sogenannten Photodisruption basiert auf sogenannter nichtlinearer Absorption. Ursprünglich transparentes Gewebe oder Material wird ab einer gewissen Laserintensitätsschwelle absorbierend. Die absorbierte Lichtenergie führt zu lokal begrenzten mikroskopischen Explosionen im Fokus des Laserstrahls. Als "ultrakurz" bezeichnet man Pulse mit einer Dauer von weniger als einer Pikosekunde ($10^{-12}$ s).

Derartige Disruptionsprozesse sind an sich bekannt, etwa aus Anwendungen wie der refraktiven Hornhaut-Chirurgie, bei der die Laserpulse Schnitte in die Hornhaut setzen, um Gewebelappen (Flaps) zu erzeugen oder Gewebe zu entfernen. Ebenfalls ist bekannt, dass ultrakurze Laserpulse nahe oder kurz unterhalb der Disruptionsschwelle das Gewebe photochemisch beeinflussen und insbesondere den optischen Brechungsindex verändern. Derartige Mechanismen wurden ebenfalls bereits verwendet, um Fehlsichtigkeiten des Auges zu beheben.

In diesen beschriebenen Fällen soll die Hornhaut des Auges nach dem Lasereingriff in optisch klar bleiben. In wenigen Einzelfällen kann sich im postoperativen Verlauf eine Narbe in der Hornhaut bilden, die zu optischen Streuphänomenen führt. Dies ist jedoch unerwünscht und es wird versucht, derartige Situationen zu vermeiden.

Aus der Katarakt-Chirurgie ist bekannt, ultrakurze Pulse zur Fragmentierung der ergrauten Augenlinse zu verwenden und/oder die Kapsel der Augenlinse zu eröffnen.

Darüber hinaus ist bekannt, ultrakurze Pulse zur Behandlung der Altersweitsichtigkeit (Presbyopie) zu verwenden, indem die ultrakurzen Pulse das mit zunehmendem Alter hart gewordene Linsenmaterial zerschneiden und dadurch die Flexibilität und Verformbarkeit der Augenlinse wieder herstellen, um die Linse beim Akkommodationsprozess wieder verformen zu können. Auch hier ist es das Ziel, die Laserpulse so zu dosieren, das nach dem Lasereingriff die Linse optisch klar bleibt, um unerwünschte Blendeffekte zu vermeiden.

Aus EP 2 231 084 B1 ist bekannt, dass man die Laserparameter so gestalten kann, dass an den durch die Laserpulse erzeugten und verbleibenden Läsionen in der Augenlinse einfallendes Licht gebeugt oder gestreut wird. Erzeugt man eine Vielzahl solcher Läsionen, kann man nach dem Prinzip der diffraktiven Optik bildformende Eigenschaften innerhalb der Augenlinse erzeugen. Mit Hilfe dieser bildformenden Eigenschaften kann man Sehfehler des Auges korrigieren.

In allen vorgenannten Anwendungsbereichen der Photodisruption am Auge bleibt der vom Laser bestrahlte Bereich entweder klar und ohne optische Wirkung oder er trägt durch Brechkraftänderung (refraktiv) oder Streuung (diffraktiv) zur Bildgebung bei. In der Kataraktchirurgie werden die vom Laser fragmentierten Linsenbestandteile operativ entfernt.

Es wurde im Rahmen der vorliegenden Erfindung realisiert, dass man bei geschickter Wahl der Laserparameter die vom Laser erzeugten Läsionen dicht genug aneinander setzen kann und damit in der Lage ist, den vom Laser bearbeiteten Bereich intransparent zu machen, ihn also als Apertur zu verwenden. Bei entsprechender Programmierung des Laserstrahls lassen sich so beispielsweise Lochblenden in der natürlichen Augenlinse erzeugen.

Diese lasergenerierte Apertur (Lochblende) kann dazu verwendet werden, nach dem Prinzip der stenopäischen Lücke (Lochblenden-Effekt) die Randstrahlen des einfallenden Lichtes abzublocken. In der Regel sind die Randstrahlen eines optischen Systems mit größeren Abbildungsfehlern (Aberrationen) behaftet. Das Ausblenden dieser Randstrahlen dient dazu, die Abbildungsqualität des Auges zu verbessern und insbesondere die Schärfentiefe des Auges zu vergrößern. Bei erhöhter Schärfentiefe ist man auch mit einem nicht-akkommodierendem (presbyopen) Auge in der Lage, Objekte in der Nähe und in der Ferne scharf zu erkennen.

In einer vorteilhaften Ausgestaltung eines Aspekts der Erfindung weist die Aperturblende laserinduzierte Läsionen in, in axialer Richtung, unterschiedlichen Ebenen auf. Mit anderen Worten ist die Steuereinheit so ausgestaltet, dass sie die Lasereinheit veranlassen kann, die laserinduzierten Läsionen in unterschiedlicher Tiefe (entlang der Sehachse des Auges) zu setzen.

Allgemein beziehen sich Angaben, wie "axial" und "lateral" auf die Sehachse des Auges, in dem die Apertur generiert wird. "Axial" beschreibt demnach eine Richtung entlang der Sehachse, "lateral" eine Richtung senkrecht zur Sehachse.

Eine möglichst vollständige Unterdrückung einer Transmission von Licht durch den Aperturbereich kann besser erreicht werden, wenn sich die Aperturblende in axialer

Richtung über mehr als eine einzelne Lage von Läsionen erstreckt. Die Aperturblende kann dabei durch eine Vielzahl von Schichten mit Läsionen aufgebaut sein, wobei hierbei die Schichten, insbesondere wenn sie in sich jeweils eine mehr oder weniger regelmäßige Verteilung der Läsionen aufweisen, lateral gegeneinander versetzt sind, etwa um jeweilige Bruchteile einer Periodizität der lateralen Verteilung der Läsionen. Eine axiale Verteilung der Läsionen, die in Form von Schichten oder dergleichen gegeben ist, ist allerdings nicht notwendig, und die Läsionen können auch in dieser Hinsicht unregelmäßig, etwa zufällig oder zumindest quasi-zufällig angeordnet sein. Hier kann man im Extremfall jeder einzelnen Läsion jeweils eine eigene Ebene zuordnen.

In einer anderen vorteilhaften Ausgestaltung eines Aspekts der Erfindung ist die Steuereinheit für eine Steuerung der Lasereinheit ausgestaltet ist, mit der aufeinanderfolgend erzeugte laserinduzierte Läsionen so erzeugt werden, dass sie voneinander in lateraler und/oder axialer Richtung wenigstens einen vorbestimmten Abstand aufweisen.

Mit dem Einbringen der laserinduzierten Läsion wird die Linse des Auges lokal gestört, insbesondere ergibt sich eine Blase im Bereich der Läsion, die sich im Laufe der Zeit wieder schließt, wobei es für die Genauigkeit und Wirksamkeit der Lasereinstrahlung von Vorteil ist, wenn der Lichtpfad nicht durch eine Läsion bzw. die noch nicht zurückgebildete Blase verläuft.

In einer anderen vorteilhaften Ausgestaltung eines Aspekts der Erfindung ist die Steuereinheit für eine Steuerung der Lasereinheit ausgestaltet, die innerhalb des Aperturbereichs zu einer zufälligen oder quasi-zufälligen Verteilung der laserinduzierten Läsionen führt.

Es kann vorgesehen sein, dass die laserinduzierten Läsionen jeweils in einer dichtest möglichen Verteilung (unter einer vereinfachenden Annahme einer kugelförmigen Blase, die bei der Erzeugung einer jeweiligen Läsion entsteht, und mit der zusätzlichen Maßgabe, dass die jeweiligen Blasenzentren um den Blasendurchmesser versetzt sind, wäre dies die dichteste Kugelpackung mit einer hexagonalen Anordnung der Läsionen). Hierbei ergibt sich allerdings eine Regelmäßigkeit der Anordnung der Läsionen, die unter Umständen zu unerwünschten optischen Effekten führen kann. Wird um den Preis einer verringerten Dichte von Läsionen diese Regelmäßigkeit durchbrochen, indem die jeweiligen Positionen der Läsionen einer zufälligen Verteilung unterliegen oder durch eine geeignete Steuerung in einer ausreichend unregelmäßigen Verteilung (also quasi-zufällig) erzeugt werden, können die optischen Effekte vermieden werden.

In einer anderen vorteilhaften Ausgestaltung eines Aspekts der Erfindung ist die Steuereinheit dazu ausgestaltet, die Lasereinheit für die Erzeugung der Aperturblende derart zu steuern, dass eine Lichttransmission durch den Aperturbereich der Linse auf 20 % oder weniger verringert wird.

Es wurde gefunden, dass für eine ausreichende Verbesserung der Sehleistung keine vollständige Unterdrückung der Transmission nötig ist und es ausreichend ist, die Lichttransmission bis einen verbleibenden Teil von 20 % oder weniger zu reduzieren. Auch wenn eine stärkere Unterdrückung der Transmission an sich von Vorteil ist und für sich genommen wünschenswert sein kann, ist der damit verbundene Aufwand in Einzelfall möglichweise zu groß, um noch durch den Zusatznutzen gerechtfertigt zu werden.

In einer anderen vorteilhaften Ausgestaltung eines Aspekts der Erfindung weist die Vorrichtung die Lasereinheit für die Erzeugung der Aperturblende in der Linse des Auges auf, wobei die Lasereinheit einen Pulslaser zur Abgabe von Laserpulsen, ein Fokussiereinheit zur Fokussierung der Laserpulse und ein Richteinheit zur Ausrichtung der Laserpulse aufweist.

Die Lasereinheit ist nicht notwendigerweise mit der Steuereinheit in einer einzigen Vorrichtung integriert, Steuereinheit und Lasereinheit können also auch getrennt voneinander vorgesehen werden, um erst bei der eigentlichen Erzeugung der Aperturblende miteinander zu kooperieren.

In einer bevorzugten Variante der obigen Ausgestaltung ist der Pulslaser zur Abgabe von Laserpulsen mit einer Pulsdauer im Bereich von 10.000 bis 10 fs, vorzugsweise von 800 bis 100 fs, besonders bevorzugt von 300 bis 150 fs, mit einer Pulsenergie im Bereich von 100 bis 0,01 $\mu$J, vorzugsweise von 10 bis 0,01 $\mu$J,

besonders bevorzugt von 2 bis 0,1 μJ, in einem Wellenlängenbereich vom 400 bis 1.400 nm, vorzugsweise von 600 bis 1.200 nm, besonders bevorzugt von 800 bis 1.100 nm und mit einer Repetitionsrate in einem Bereich von 1 bis 100.000 kHz, vorzugsweise von 10 bis 10.000 kHz, besonders bevorzugt von 100 bis 500 kHz ausgestaltet, ganz besonders bevorzugt mit einer Pulsdauer von 150 fs, einer Pulsenergie von 1 μJ, einer Wellenlänge im Bereich von 700 bis 1.100 nm und einer Repetitionsrate von 200 kHz.

Gemäß der Lehre von EP 2 231 084 B1 sind Laserpulse mit einer Pulsdauer von 100 fs, einer Pulsenergie von 1 μJ, einer Wellenlänge von 700 bis 1100 nm und einer Repetitionsrate von 100 kHz typisch, um eine permanente Läsion in der natürlichen Augenlinse zu erzeugen. Im Gegensatz zum Ansatz der Lehre von EP 2 231 084 B1 ist das Ziel dieser Erfindung jedoch nicht, an den definiert beabstandeten Laserläsionen singuläre Streu-Ereignisse zu erzeugen, die in Summe in Transmission durch die Augenlinse eine Bildgebung bewirken. Vielmehr soll erfindungsgemäß im Bereich der erzeugten Laserläsionen möglichst wenig Licht in Richtung Netzhaut transmittieren und vorzugsweise keine Transmission in Richtung Netzhaut stattfinden.

Erfindungsgemäß wird nach einem weiteren Aspekt ein Computerprogramm mit Programmmitteln, die eine erfindungsgemäße Vorrichtung dazu veranlassen, ein Verfahren zur Erzeugung einer Aperturblende in einem Auge auszuführen, wenn das Computerprogramm auf der Vorrichtung ausgeführt wird. Das Computerprogramm kann auf einem geeigneten Speichermedium, etwa einem optischen Speichermedium oder einem nichtflüchtigen elektronischen Speichermedium, vorgesehen, gespeichert und/oder vertrieben werden. Es kann auch zusammen mit oder als Teil einer Hardware-Komponente bereitgestellt werden. Das Computerprogramm kann auch auf andere Weise bereitgestellt werden, etwa über das Internet oder über drahtgebundene oder drahtlose Telekommunikationsmittel.

Der Umfang der Erfindung wird durch die Ansprüche definiert.

Merkmale vorteilhafter Ausführungsformen der Erfindung sind insbesondere in den Unteransprüchen definiert, wobei weitere vorteilhafte Merkmale, Ausführungen und Ausgestaltungen für den Fachmann zudem aus den obigen Erläuterung und der folgenden Diskussion zu entnehmen sind.

Im Folgenden wird die vorliegende Erfindung anhand von in den Figuren dargestellten Ausführungsbeispielen weiter illustriert und erläutert. Hierbei zeigt

Fig. 1 schematische Darstellungen einer Aperturblende in einer natürlichen Linse des Auges,

Fig. 2 eine schematische Darstellung zur Erläuterung der Lichtverhältnisse bei einer Linse mit der Aperturblende und der Iris des Auges,

Fig. 3 schematische Darstellungen zum Vergleich einer Transmission jeweils mit einer einlagigen Anordnung von Läsionen und einer mehrschichtigen Anordnung von Läsionen,

Fig. 4 schematische Illustrationen der Vorgänge bei einer Photodisruption,

Fig. 5 schematische Illustrationen in einem Fall einer zu engen Positionierung von Laserpulsen,

Fig. 6 schematische Darstellungen zur Orientierung der Aperturblende zur optischen Achse,

Fig. 7 schematische Darstellungen einer axialen Anordnung von laserinduzierten Läsionen und

Fig. 8 schematische Darstellungen zur Erläuterung von Abläufen bei der Erstellung einer erfindungsgemäßen Aperturblende mit vorheriger Markierung.

In den beiliegenden Zeichnungen sowie den Erläuterungen zu diesen Zeichnungen sind einander entsprechende bzw. in Beziehung stehende Elemente - soweit zweckdienlich - mit jeweils entsprechenden oder ähnlichen Bezugszeichen gekennzeichnet, auch wenn sie in unterschiedlichen Ausführungsbeispielen zu finden sind.

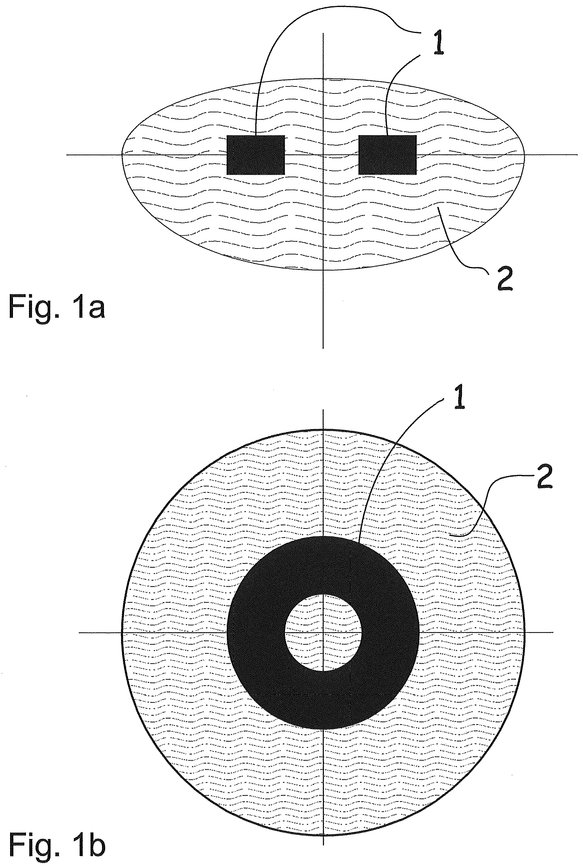

Fig. 1a zeigt eine beispielhafte Seitenansicht einer Positionierung einer lasererzeugten Apertur 1 innerhalb einer natürlichen Linse 2 eines Auges, wobei Fig. 1b eine entsprechende Draufsicht der Positionierung der lasererzeugten Apertur 1 innerhalb der natürlichen Linse 2 des Auges zeigt. Die Sehachse und axiale Richtung ist in dieser Figur als senkrechte Linie eingezeichnet, eine laterale Richtung als horizontale Linie.

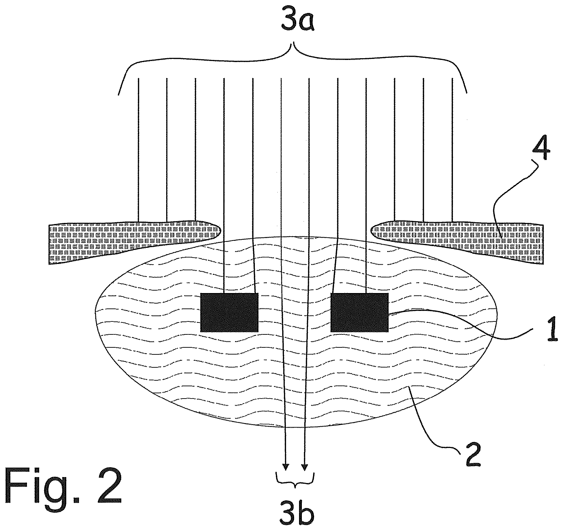

Fig. 2 zeigt eine schematische Darstellung zur Erläuterung der Lichtverhältnisse bei einer Linse mit der Aperturblende und der Iris des Auges. In das Auge einfallendes Licht 3a wird zum einen in natürlicher Weise von der Iris 4 des Auges abgeblockt und darüber hinaus von der lasererzeugten Apertur 1 innerhalb der Linse 2. Nur der zentrale Teil 3b des Lichts 3a wird hierbei bis zur Netzhaut durchgelassen.

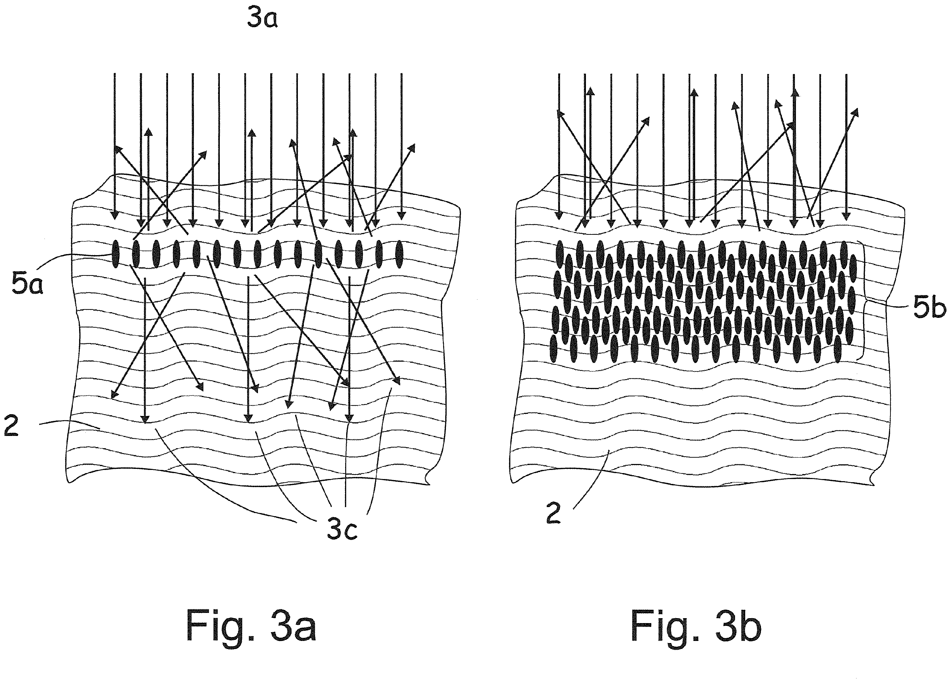

Fig. 3 zeigt schematische Darstellungen zum Vergleich einer Transmission jeweils mit einer einlagigen Anordnung von Läsionen und einer mehrschichtigen Anordnung von Läsionen. Eine einlagige Anordnung von Laserläsionen 5a innerhalb der Linse 2 des Auges kann, wie in diesem Beispiel, dazu führen, dass einfallendes Licht 3a vorwiegend in alle Richtungen gestreut wird. Selbst bei sehr dichter lateraler Anordnung der Läsionen 5a kann es vorkommen, dass ein noch immer erheblicher Teil des einfallenden Lichtes vorwärts gestreut (3c) wird und somit die Netzhaut erreichen kann. Im Gegensatz dazu vermindern, wie in Fig. 3b illustriert, mehrere Schichten lateral dicht gepackter Läsionen 5b sehr weitgehend die Wahrscheinlichkeit, dass Photonen in Richtung Netzhaut gestreut werden.

Wenn, wie im Rahmen der vorliegenden Erfindung vorgesehen, möglichst wenig Licht durch den Aperturbereich (der die Aperturöffnung umgibt) hindurchtreten soll, sind die Laserläsionen lateral möglichst dicht anzuordnen. Da die Laserläsionen zum Teil das Licht nicht absorbieren, sondern lediglich streuen, kann es dennoch, selbst bei sehr dichter lateraler Anordnung der Läsionen, dazu kommen, dass ein Teil des einfallenden Lichtes vorwärts gestreut und somit die Netzhaut erreichen (Fig. 3a). Die gestreuten Photonen, die die Netzhaut erreichen, tragen nicht zur Bildentstehung bei und werden als störend empfunden. Insbesondere das Kontrastsehen wird dadurch negativ beeinflusst.

Erzeugt man hingegen auch in axialer Richtung mehrere Schichten lateral dicht gepackter Läsionen, so wird die Wahrscheinlichkeit, dass Photonen in Richtung Netzhaut gestreut werden, zunehmend geringer. Ab einer bestimmten Dicke bzw. Anzahl hintereinander liegender Schichten ist auch ohne weitere besondere Maßnahmen hinsichtlich der Ausgestaltungen der Läsionen selbst die Menge an transmittiertem Licht so gering, dass der Kontrast bei der Bildentstehung auf der Netzhaut nur unwesentlich oder akzeptabel verringert wird (Fig. 3b).

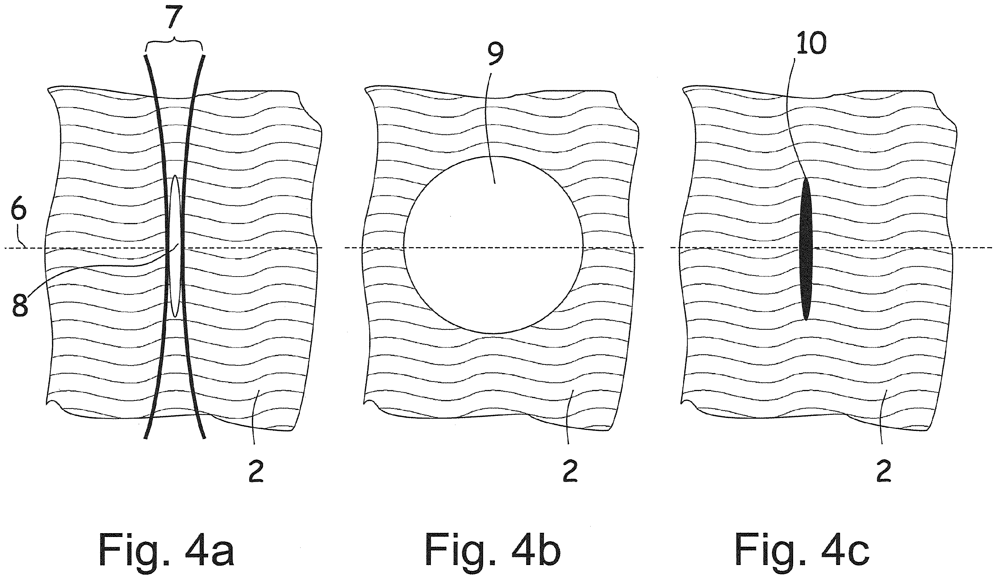

Fig. 4 zeigt schematische Illustrationen der Vorgänge bei einer Photodisruption.

Fig. 4a illustriert, dass in der Fokalebene 6 des einfallenden Laserstrahls 7 bei hinreichender Intensität des Laserstrahls eine Photodisruption stattfindet. Physikalisch bedingt hat die Wechselwirkungszone 8 typischerweise eine prolate Form.

Fig. 4b illustriert, dass unmittelbar nach der Disruption durch das verdampfende Linsenmaterial eine Gasblase 9 mit erheblich größerer Ausdehnung als die Wechselwirkungszone 8 des Laserlichts entsteht.

Nach einer bestimmten Zeit (Millisekunden bis Stunden), abhängig von der Größe der Gasblase, ist das Gas in die Umgebung des Linsengewebes diffundiert und die Gasblase ist kollabiert, wie es in Fig. 4c dargestellt ist. Zurück bleibt eine prolate Form von verändertem Linsengewebe (hier "Laserläsion" 10 genannt). Die Laserläsion 10 zeichnet sich durch stark streuende und absorbierende Eigenschaften für sichtbares Licht aus.

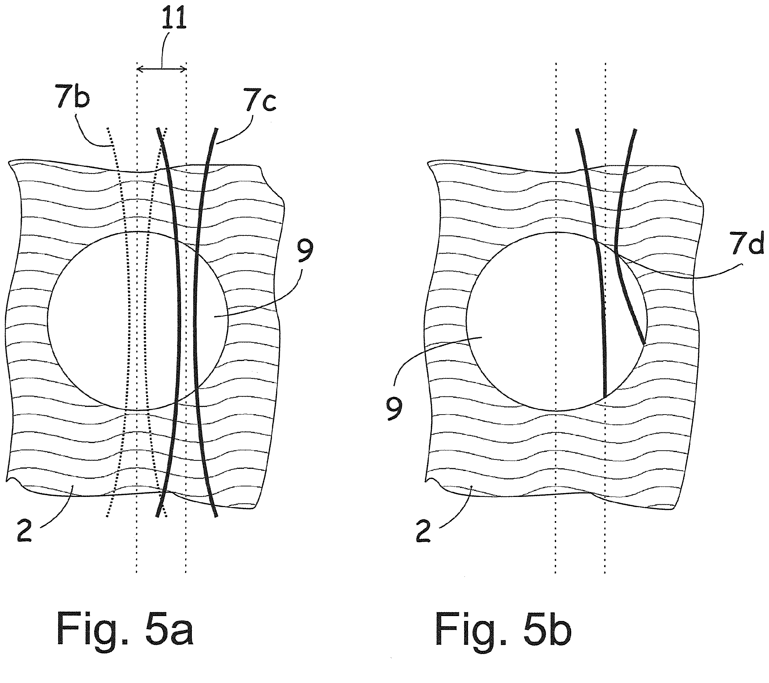

Fig. 5 zeigt schematische Illustrationen in einem Fall einer zu engen Positionierung von Laserpulsen. Haben zwei zeitlich aufeinander folgende Laserpulse 7b, 7c einen räumlichen Abstand 11 kleiner als der Durchmesser der verbliebenen Gasblase 9, wird, wie es in Fig. 5a gezeigt ist, der Folgepuls 7c in die Gasblase 9 gelenkt. Trifft der Laserpuls innerhalb der Linse 2 auf die Gasblase 9, wird aufgrund der Brechungsunterschiede zwischen Linsenmaterial und Gasblase das Licht des Laserpulses 7d abgelenkt, wie es in Fig. 5b illustriert ist.

Vorzugsweise werden die Laserparameter so eingestellt, dass jeweils in der Fokusebene des Laserpulses die Schwelle zur Photodisruption überschritten wird, um eine permanente Läsion zu erzeugen (Fig. 4a). Dies hat jedoch zur Folge, dass unmittelbar nach Lasereinwirkung am Fokus des Laserpulses eine kleine Gasblase erzeugt wird (Fig. 4b). Das Gas ist das Produkt des vom Laserpuls verdampften Wassers oder in die Gasphase überführten Linsenbestandteile. Aufgrund der Viskosität und der chemischen Löslichkeit der erzeugten Gase und Dämpfe im Gewebewasser dauert es eine gewisse Zeit, bis die Blase vollständig kollabiert ist und eine typischerweise ellipsenförmige, prolate Läsion übrigbleibt, die dauerhaft das einfallende Licht streut oder absorbiert (Fig. 4c). Gewöhnlich ist die Zeitdauer, bis die Gasblase kollabiert, wesentlich größer (Millisekunden bis Stunden) als der zeitliche Abstand zweier Laserpulse (Nanosekunden bis Millisekunden). Wird der Laser so gesteuert, dass ein Folgepuls in einem Abstand kleiner als der Durchmesser der verbliebenen Gasblase platziert wird, wird der Folgepuls in die Gasblase gelenkt (Fig. 5a). In dieser Situation wird aufgrund der Brechungsunterschiede zwischen Linsenmaterial und Gasblase das Licht des Laserpulses abgelenkt (Fig. 5b). Dabei kann die Fokusqualität des Laserpulses zerstört werden, so dass die Lichtintensität im Fokus nicht mehr ausreichend aufrechterhalten werden kann, um die Schwelle für einen Photodisruptions-Prozess zu überschreiten. Je nach Größe der Gasblase und zeitlichem und räumlichem Abstand der Laserpulse können mehrere Folgepulse an einer Gasblase abgelenkt werden und keine Photodisruption erzeugen. In der Folge entstehen so Muster von Läsionen die viel größere Abstände aufweisen als die ursprünglich beabsichtigten Muster der Laserfoki. Folglich ist die Abschottungswirkung der gesamten Laserbereiche weniger effizient.

Diese Effizienzminderung lässt sich umgehen, indem die Lasersteuerung so programmiert wird, dass zeitlich aufeinanderfolgende Laserpulse einen größeren Abstand haben als der Durchmesser der Gasblasen zu dem Zeitpunkt, an dem der Folgepuls eintrifft.

Die verbleibenden Lücken zwischen den Laserläsionen können in einem zweiten und / oder vielfachen Durchlauf von Laserpulsen geschlossen werden, sobald die Gasblasen kollabiert sind.

Dauert der Kollaps der Gasblasen länger als das Abrastern der gesamten Fläche einer Ebene von Laserläsionen, so kann der Laser so programmiert werden dass er in einer weiteren, der Laserquelle zugewandten Richtung eine weitere Lage von Laserläsionen erzeugt. Vorzugsweise sind die Laserläsionen dann an den Lücken platziert, die von den Laserläsionen in der Ebene zuvor zurückgelassen wurden. Dieser Prozess kann mehrfach wiederholt werden und dadurch einen Volumenkörper erzeugen, der ab einer gewissen Anzahl von Lagen an Laserläsionen hinreichend wenig Licht zur Netzhaut durchdringen lässt.

In einer weiteren Ausführungsform können die Laserläsionen im Mittel zufällig beabstandet werden. Durch die unregelmäßig räumliche Verteilung der Laserläsionen wird verhindert, dass die Laserläsionen ein optisches Gitter erzeugen und dabei unerwünschte Beugungseffekte hervorrufen.

Wird die lasergenerierte Apertur durch mehrere Lagen von Laserläsionen erzeugt, stellt die Apertur ein räumliches Objekt dar. Folglich ist neben der Zentrierung der Apertur beispielsweise zur Sehachse des Auges nicht nur die laterale Ausrichtung der Apertur von Bedeutung, sondern auch die Orientierung. Bei schiefer Orientierung wirkt die kreisrunde Öffnung der Apertur in Projektion zur Sehachse bzw. zum einfallenden Licht als Ellipse. Darüber hinaus wird das Licht an den Kanten der Öffnung starke Streuphänomene hervorrufen und eine Blendwirkung verursachen.

Aus diesem Grund ist es vorteilhaft, die axiale Ausdehnung (in Strahlrichtung) der Apertur so gering wie möglich zu halten und dennoch möglichst viele Lagen von Laserläsionen übereinander zu legen.

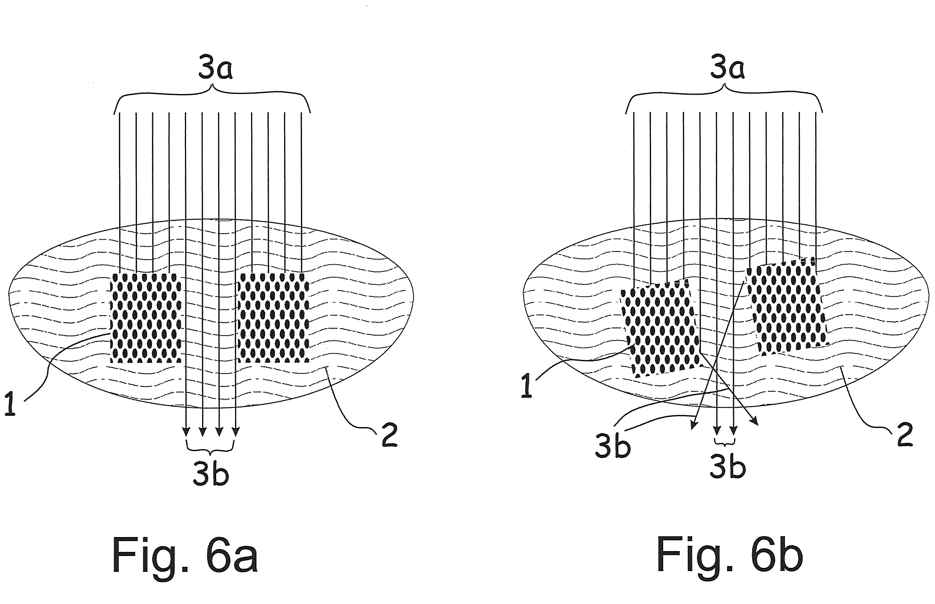

Fig. 6a illustriert, dass bei korrekter Orientierung der Apertur 1 in der Linse 2 parallel zur optischen Achse bzw. des einfallenden Lichts 3a die zentralen Strahlen des Lichtbündels 3a durchgelassen und die Randstrahlen durch Streuung bzw. Absorption abgeblockt werden.

Fig. 6b illustriert, dass bei schiefer Orientierung der Apertur das einfallende Licht 3a an den Kanten der Öffnung der Apertur Streuphänomene hervorruft. Lichtstrahlen 3c, die auf die Kanten der Apertur treffen werden, nur leicht gestreut und können die Netzhaut erreichen. Sie verursachen dadurch eine unerwünschte Blendwirkung.

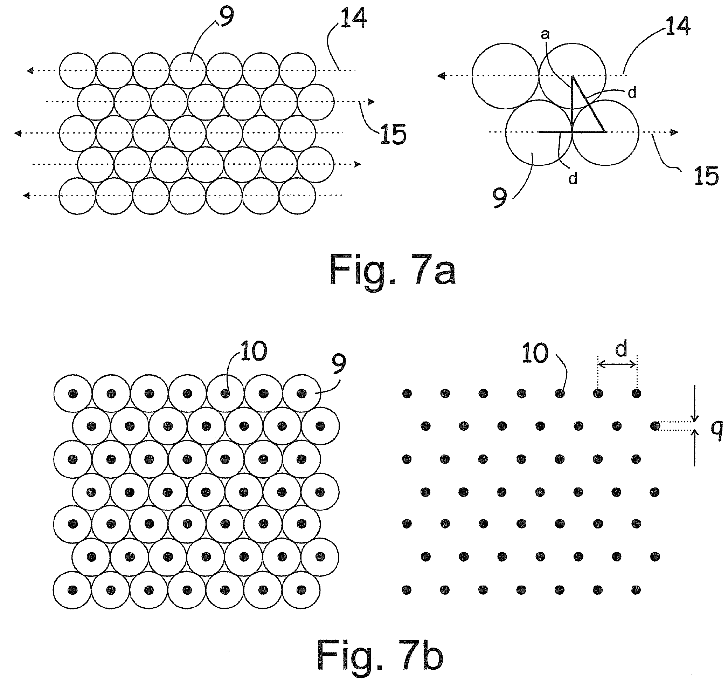

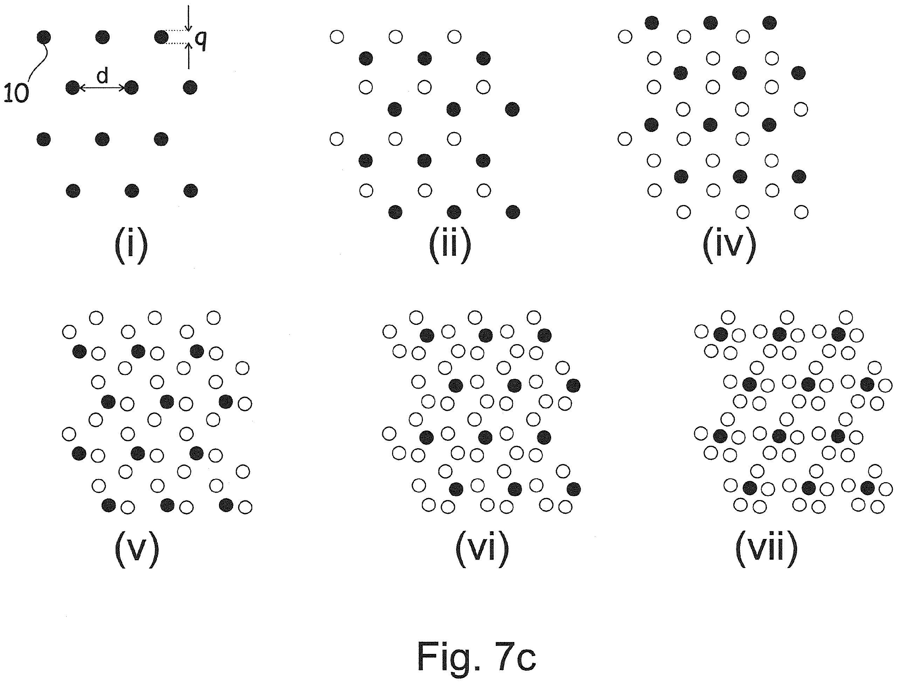

Fig. 7 zeigt schematische Darstellungen einer axialen Anordnung von laserinduzierten Läsionen.

Eine möglichst dichte Anordnung der Laserläsionen bekommt man, wenn man die Laserpulse in einer Richtung gerade so weit beabstandet, wie sich eine lasererzeugte Gasblase im Linsengewebe maximal ausdehnt. Folglich entsteht eine Kette von einzelnen Gasblasen, die sich berühren, vorzugsweise aber nicht miteinander verschmelzen. (Fig. 7a). In einer benachbarten Strecke 15, parallel zur ersten Strecke 14, werden die einzelnen Laserpulse um die Hälfte des maximalen Gasblasendurchmesser d versetzt angeordnet. Der minimale Abstand a der benachbarten Strecke berechnet sich zu:

$$a = \sqrt{3/4} \times d$$

Nach dem vollständigen Abrastern einer Ebene ergibt sich ein Teppich von Läsionen 10, die im Abstand d voneinander getrennt liegen. Die Läsionen 10 und damit die für das einfallende Licht 3a wirksame, absorbierende bzw. streuende Kreisfläche haben den Durchmesser q. Folglich kann eine solche Ebene von Laserläsionen nur den Bruchteil $(q/d)^2$ des einfallenden Lichtes streuen bzw. absorbieren (Fig. 7b).

Hier kann nun eine zweite Ebene von Laserläsionen erzeugt werden. Diese Ebene liegt in der dem Laserstrahl zugewandten Richtung und hat den Abstand a zur vorherigen Ebene. Für den Fall, dass sich während der Erzeugung einer Ebene von Läsionen die Gasblasen in der Ebene verkleinert haben, kann der Abstand auch geringer gewählt werden. In lateraler Ausrichtung kann das Raster der Laserpulse so versetzt werden, dass die Läsionen der Laserpulse der neueren Lage zwischen den Läsionen der vorherigen Lage liegen. In einer folgenden, dritten Lage, im Abstand a oder kleiner von der zweiten Lage kann abermals ein Raster von Läsionen erzeugt werden. In diesem Fall werden die Laserfoki und die sich daraus ergebenden Läsionen in die Projektion der noch offenen Lücken aller vorherigen Läsionen platziert. Die Abfolge der Erzeugung der einzelnen Lagen von Läsionen in axial unterschiedlichen Ebenen ist in Fig.7c für die ersten sechs Lagen beispielhaft dargestellt, Dabei sind für die aktuell erzeugten Läsionen schwarze Vollkreise verwendet worden und für die jeweils vorherigen Lagen offene weiße Kreise.

Diese Abfolge kann so oft wiederholt werden, bis in Summe eine hinreichende Anzahl von Läsionen erzeugt wurde, die die gesamte Fläche der Apertur bedecken. Will man beispielsweise 100% der Aperturfläche mit Läsionen abdecken, so sind mindestens $(d/q)^2$ Lagen an Laserläsionen zu erzeugen.

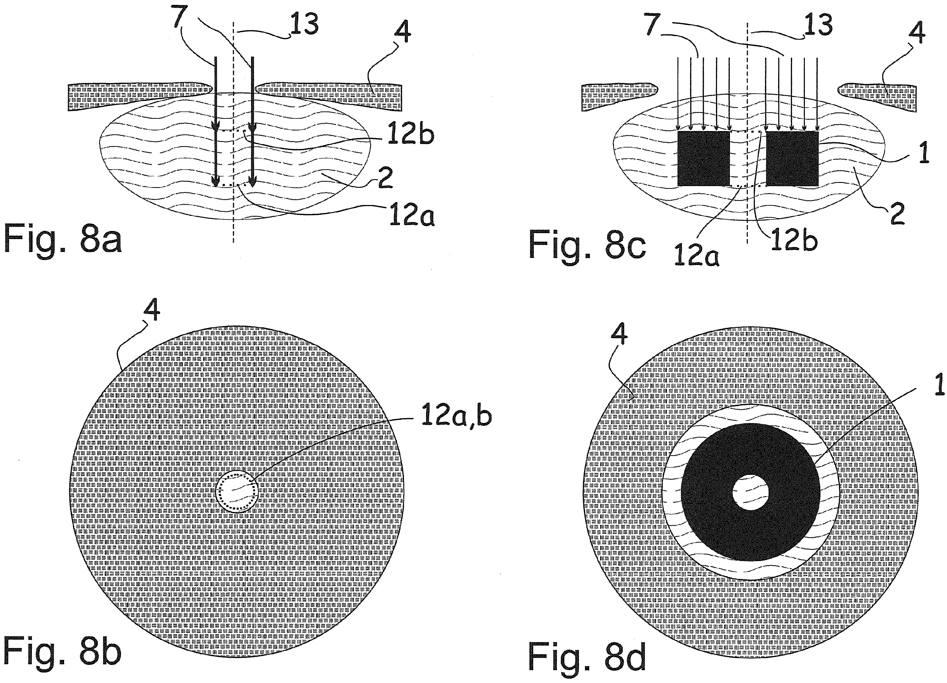

Fig. 8 zeigt schematische Darstellungen zur Erläuterung von Abläufen bei der Erstellung einer erfindungsgemäßen Aperturblende mit vorheriger Markierung.

Bei verengter Pupille (Miosis) können mit Hilfe des Laserstrahls 7 ein oder mehrere Orientierungspunkte 12a, 12b in die Linse 2 gelasert werden, wie es in Fig. 8a gezeigt ist. Beispielsweise können dies zwei Ringe sein, die die hintere (12a) und vordere Position (12b) der Apertur-Öffnung andeuten. Die laterale Position der Markierungen wird dabei durch den inneren Rand der Iris 4 begrenzt. Fig. 8b zeigt die gleiche Situation wie in Fig. 8a aus der Sicht des Operateurs (Draufsicht)

Fig. 8c zeigt, dass bei weiter Pupillenstellung (Mydriasis), ohne dass die Iris 4 den Strahlengang des Lasers 7 abschattet, die vollständige Apertur 1 in die Linse 2 gelasert werden kann. Dabei helfen die zuvor gesetzten Markierungen 12a, 12b, die Apertur entsprechend zu zentrieren und entlang der Sehachse 13 zu orientieren. Fig. 8d zeigt die gleiche Situation wie in Fig. 8c aus der Sicht des Operateurs (Draufsicht).

Die lasergenerierte Apertur kann zur Sehachse des Auges zentriert und orientiert werden. Beispielsweise ist vor von Vorteil, das Zentrum der Apertur in die Mitte zwischen dem Pupillenzentrum und dem sog. ersten Purkinje-Reflex zu positionieren. Wenn die Pupille bei der Laseranwendung medikamentös erweitert werden muss (Mydriasis), ist es vorteilhaft, wenn zunächst bei enger Pupille eine Markierung auf der Hornhautoberfläche, beispielsweise durch Farbe, angebracht wird.

Eine vorteilhafte Positionierung ergibt sich durch die Zentrierung und Orientierung an der Iris 4 bei verengter Pupille (Miosis). Nachdem das Patientenauge auf die Laserapparatur ausgerichtet wurde und ggf. fixiert wurde, beispielsweise durch ein übliches sog. Patienten-Interface, wird das Auge durch vergleichsweise helles Licht gereizt. Folglich adaptiert das Auge durch eine Pupillenengstellung (Miosis). In dieser Situation können vom Laser 7 ein oder mehrere Orientierungspunkte 12a, 12b in die Linse 2 gelasert werden, beispielsweise zwei Ringe, die die hintere und vordere Position der Apertur-Öffnung andeuten (Fig. 8a, 8b). Die Positionierung kann durch übliche Bildgebung, beispielsweise Optische Kohärenztomographie (OCT) unterstützt werden.

Nach erfolgter Markierung wird der Lichtreiz entfernt. Vorzugsweise wird das Auge, bzw. werden beide Patientenaugen maximaler Dunkelheit überlassen. Folglich wird das Auge erneut die Lichtsituation adaptieren und die Pupille weitstellen (Mydriasis). In dieser Situation kann nun, ohne dass die Iris 4 den Strahlengang des Lasers abschattet, die vollständige Apertur 1 in die Linse 2 gelasert werden. Dabei helfen die zuvor gesetzten Markierungen 12a, 12b, die Apertur entsprechend zu zentrieren und entlang der Sehachse 13 zu orientieren. Reicht die natürliche Mydriasis nicht aus, um die Iris 4 vollständig aus dem Strahlengang des Lasers 7 zu bewegen kann die Mydriasis medikamentös erweitert werden. Diese Vorgehensweise hat den Vorteil, dass nach dem Lasereingriff, bei hellem Tageslicht, die natürlicherweise eng adaptierte Pupille mit der Öffnung der lasergenerierten Apertur übereinstimmt.

Auch wenn in den Figuren verschiedene Aspekte oder Merkmale der Erfindung jeweils in Kombination gezeigt sind, ist für den Fachmann - soweit nicht anders angegeben - ersichtlich, dass die dargestellten und diskutieren Kombinationen nicht die einzig möglichen sind. Insbesondere können einander entsprechende Einheiten oder Merkmalskomplexe aus unterschiedlichen Ausführungsbeispielen miteinander ausgetauscht werden.

In Implementierungen der Erfindung können jeweils einzelne Komponenten, z.B. ein Prozessor, ganz oder teilweise die Funktionen verschiedener in den Ansprüchen genannter Elemente übernehmen. Abläufe oder Vorgänge wie beispielsweise Steuerungen, Berechnungen, Erfassungen oder ähnliches können als Programmmittel eines Computerprogramms und/oder als spezielle Hardwarekomponenten implementiert werden.

**Patentansprüche**

1. Vorrichtung zur Erzeugung einer Aperturblende (1) in einem Auge, mit:

    einer Steuereinheit für eine Lasereinheit, wobei die Steuereinheit dazu ausgestaltet ist, die Lasereinheit für eine Erzeugung der Aperturblende (1) in einer Linse (2) des Auges zu steuern, wobei die Aperturblende (1) zur Vergrößerung der Schärfentiefe des Auges dient und durch laserinduzierte Läsionen (5a, 5b, 10) gebildet ist, die eine Lichttransmission durch einen eine Aperturöffnung umgebenden Aperturbereich der Linse (2) verringern, **dadurch gekennzeichnet, dass**

die Vorrichtung ferner eine Ausrichtungseinheit zur Ausrichtung und/oder Fixierung des Auges und eine Lichtreizeinheit zur Lichtreizung des Auges aufweist, um eine Pupillenengstellung zu bewirken, wobei die Steuereinheit ausgestaltet ist, die Lasereinheit anhand der Pupillenengstellung zur Erstellung von Markierungen (12a, 12b) zu steuern, die die Aperturöffnung in lateraler und/oder axialer Richtung (13) definieren.

2. Vorrichtung nach Anspruch 1, wobei die Aperturblende (1) laserinduzierte Läsionen (5a, 5b, 10) in, in axialer Richtung (13), unterschiedlichen Ebenen aufweist.

3. Vorrichtung nach einem der vorstehenden Ansprüche, wobei die Steuereinheit für eine Steuerung der Lasereinheit ausgestaltet ist, mit der aufeinanderfolgend erzeugte laserinduzierte Läsionen (5a, 5b, 10) so erzeugt werden, dass sie voneinander in lateraler und/oder axialer Richtung (13) wenigstens einen vorbestimmten Abstand (d) aufweisen.

4. Vorrichtung nach Anspruch 3, wobei die Steuereinheit für eine Steuerung der Lasereinheit ausgestaltet ist, so dass zeitlich aufeinanderfolgende Laserpulse (7, 7b, 7c, 7d) zur Erzeugung der laserinduzierten Läsionen (5a, 5b, 10) einen größeren Abstand haben als der Durchmesser der Gasblasen (9) zu dem Zeitpunkt, an dem der Folgepuls eintrifft, wobei die Steuereinheit ferner dazu ausgestaltet ist, die Lasereinheit so zu steuern, dass dabei verbleibende Lücken zwischen den laserinduzierten Läsionen (5a, 5b, 10) in einen folgenden Durchgang geschlossen werden, nachdem die Gasblasen (9) kollabiert sind.

5. Vorrichtung nach einem der vorstehenden Ansprüche, wobei die Steuereinheit für eine Steuerung der Lasereinheit ausgestaltet ist, die innerhalb des Aperturbereichs zu einer zufälligen oder quasi-zufälligen Verteilung der laserinduzierten Läsionen (5a, 5b, 10) führt.

6. Vorrichtung nach einem der vorstehenden Ansprüche, wobei die Steuereinheit dazu ausgestaltet ist, die Lasereinheit für die Erzeugung der Aperturblende (1) derart zu steuern, dass eine Lichttransmission durch den Aperturbereich der Linse (2) auf 20 % oder weniger verringert wird.

7. Vorrichtung nach einem der vorstehenden Ansprüche, mit der Lasereinheit für die Erzeugung der Aperturblende (1) in der Linse (2) des Auges, wobei die Lasereinheit einen Pulslaser zur Abgabe von Laserpulsen (7, 7b, 7c, 7d), ein Fokussiereinheit zur Fokussierung der Laserpulse (7, 7b, 7c, 7d) und ein Richteinheit zur Ausrichtung der Laserpulse (7, 7b, 7c, 7d) aufweist.

8. Vorrichtung nach Anspruch 7,

   wobei der Pulslaser zur Abgabe von Laserpulsen (7, 7b, 7c, 7d) mit einer Pulsdauer im Bereich von 10.000 bis 10 fs, vorzugsweise von 800 bis 100 fs, besonders bevorzugt von 350 bis 150 fs,
   mit einer Pulsenergie im Bereich von 100 bis 0,01 μJ, vorzugsweise von 10 bis 0,1 μJ, besonders bevorzugt von 2 bis 0,1 μJ,
   in einem Wellenlängenbereich vom 400 bis 1.400 nm, vorzugsweise von 600 bis 1.200 nm, besonders bevorzugt von 800 bis 1.100 nm und
   mit einer Repetitionsrate in einem Bereich von 1 bis 100.000 kHz, vorzugsweise von 10 bis 10.000 kHz, besonders bevorzugt von 100 bis 500 kHz ausgestaltet ist,
   ganz besonders bevorzugt mit einer Pulsdauer von 150 fs, einer Pulsenergie von 1 μJ, einer Wellenlänge im Bereich von 700 bis 1100 nm und einer Repetitionsrate von 200 kHz.

9. Verfahren zur Erzeugung von Steuerbefehlen für eine Lasereinheit zur Erzeugung einer Aperturblende (1) in einem Auge, wobei die Steuerbefehle die Lasereinheit zu einer Erzeugung der Aperturblende (1) in einer Linse (2) des Auges veranlassen, wobei die Aperturblende (1) zur Vergrößerung der Schärfentiefe des Auges dient und durch laserinduzierte Läsionen (5a, 5b, 10) gebildet ist, die eine Lichttransmission durch einen eine Aperturöffnung umgebenden Aperturbereich der Linse (2) verringern, **dadurch gekennzeichnet, dass**
   die Steuerbefehle die Lasereinheit im Zusammenspiel mit einer Ausrichtungseinheit zur Ausrichtung und/oder Fixierung des Auges veranlassen, anhand einer durch eine Lichtreizeinheit zur Lichtreizung des Auges zu Bewirkung einer Pupillenengstellung bewirkte Pupillenengstellung Markierungen (12a, 12b) zu erstellen, die die Aperturöffnung in lateraler und/oder axialer Richtung (13) definieren.

10. Computerprogramm mit Programmmitteln, die eine Vorrichtung nach Anspruch 1 dazu veranlassen, ein Verfahren zur Erzeugung einer Aperturblende (1) in einem Auge auszuführen, wenn das Computerprogramm auf der Vorrichtung ausgeführt wird.

**Claims**

1. A device for creating an aperture (1) in an eye, comprising:

   a control unit for a laser unit,
   wherein the control unit is configured to control the laser unit to create the aperture (1) in a lens (2) of the eye, wherein the aperture (1) is used to increase the depth of field of the eye and is formed by laser-induced lesions (5a, 5b, 10) which reduce light transmission through an aperture region of the lens (2) surrounding an aperture opening,
   **characterized in that**
   the device further comprises an alignment unit for aligning and/or fixating the eye and a light stimulation unit for light stimulation of the eye in order to narrow the pupil, wherein the control unit is configured to control the laser unit on the basis of the narrowed pupil in order to produce marks (12a, 12b) defining the aperture opening in the lateral and/or axial direction (13).

2. The device according to claim 1, wherein the aperture (1) has laser-induced lesions (5a, 5b, 10) in different planes in the axial direction.

3. The device according to any one of the preceding claims, wherein the control unit is configured to control the laser unit with which laser-induced lesions (5a, 5b, 10) are sequentially created in such a way that they are spaced apart from each other in the lateral and/or axial direction (13) by at least a predetermined distance (d).

4. The device according to claim 3, wherein the control unit is configured to control the laser unit with which time-wise sequential laser pulses (7, 7b, 7c, 7d) for forming the laser-induced lesions (5a, 5b, 10) are spaced apart by a distance which is bigger than the diameter of the gas bubbles (9) at the moment in time when the subsequent pulse arrives, wherein the control unit is further configured to control the laser unit such that remaining gaps between the laser-induced lesions (5a, 5b, 10) are closed in a following sequence, once the gas bubbles have collapsed.

5. The device according to any one of the preceding claims, wherein the control unit is configured to control the laser unit in a way which results in a random or semi-random distribution of the laser-induced lesions (5a, 5b, 10) within the aperture region.

6. The device according to any one of the preceding claims,

wherein the control unit is configured to control the laser unit for creating the aperture (1) in such a way that light transmission through the lens aperture region is reduced to 20% or less.

7. The device according to any one of the preceding claims,

comprising the laser unit for creating the aperture (1) in the lens (2) of the eye, wherein the laser unit has a pulsed laser for emitting laser pulses (7, 7b, 7c, 7d), a focusing unit for focusing the laser pulses (7, 7b, 7c, 7d), and an alignment unit for aligning the laser pulses (7, 7b, 7c, 7d).

8. The device according to claim 7,

wherein the pulsed laser is configured to emit laser pulses (7, 7b, 7c, 7d)

having a pulse duration in the range between 10,000 to 10 fs, preferably in the range between 800 and 100 fs, particularly preferably in the range between 350 and 150 fs,

with a pulse energy in the range between 100 and 0.01 $\mu$J, preferably in the range between 10 and 0.1 $\mu$J, particularly preferably in the range between 2 and 0.1 $\mu$J,

in the wavelength range between 400 and 1,400 nm, preferably in the range between 600 and 1,200 nm, particularly preferably in the range between 800 and 1,100 nm and

with a repetition rate in the range between 1 and 100,000 kHz, preferably in the range between 10 and 10,000 kHz, particularly preferably in the range between 100 and 500 kHz,

especially preferably with a pulse duration of 150 fs, a pulse energy of 1 $\mu$J, a wavelength in the range between 700 and 1100 nm and a repetition rate of 200 kHz.

9. A method of generating control commands for a laser unit for creating an aperture (1) in an eye, wherein the control commands cause the laser unit to create the aperture (1) in a lens (2) of the eye, wherein the aperture (1) is used to increase the depth of field of the eye and is formed by laser-induced lesions (5a, 5b, 10) which reduce light transmission through an aperture region of the lens (2) surrounding an aperture opening,

**characterized in that**

the control commands cause the laser unit, in cooperation with an alignment unit for aligning and/or fixating the eye, based on a narrowed pupil, which is effected by a a light stimulation unit for light stimulation of the eye in order to narrow the pupil, to produce marks (12a, 12b) defining the aperture opening in the lateral and/or axial direction (13).

10. A computer program, comprising programming means which cause a device according to claim 1 to perform a method of creating an aperture (1) in an eye when the computer program is executed on the device.

**Revendications**

1. Dispositif de génération d'un diaphragme d'ouverture (1) dans un œil, avec :

une unité de commande pour une unité laser, dans lequel l'unité de commande est configurée pour commander l'unité laser pour la génération du diaphragme d'ouverture (1) dans un cristallin (2) d'un œil, dans lequel le diaphragme d'ouverture (1) sert à augmenter la profondeur de champ de l'œil et est formé par des lésions induites par laser (5a, 5b, 10) qui réduisent une transmission de lumière à travers une zone d'ouverture du cristallin (2) entourant une ouverture, **caractérisé en ce que** le dispositif présente en outre une unité d'alignement pour l'alignement et/ou la fixation de l'œil et une unité d'excitation de la lumière pour l'excitation par la lumière de l'œil pour provoquer un rétrécissement de la pupille, dans lequel l'unité de commande est configurée pour commander l'unité laser à l'aide du rétrécissement de la pupille pour créer des repères (12a, 12b) qui définissent l'ouverture dans une direction latérale et/ou axiale (13).

2. Dispositif selon la revendication 1, dans lequel le diaphragme d'ouverture (1) présente des lésions induites par laser (5a, 5b, 10) dans des plans différents, dans la direction axiale (13).

3. Dispositif selon l'une quelconque des revendications précédentes, dans lequel l'unité de commande est configurée pour commander l'unité laser avec laquelle des lésions induites par laser (5a, 5b, 10) générées successivement sont générées de telle sorte qu'elles présentent au moins une distance (d) prédéterminée les unes des autres dans la direction latérale et/ou axiale (13).

4. Dispositif selon la revendication 3, dans lequel l'unité de commande est configurée pour commander l'unité laser de telle sorte que des impulsions laser successives dans le temps (7, 7b, 7c, 7d) pour générer les lésions induites par laser (5a, 5b, 10) présentent un plus grand espacement que le diamètre des bulles de gaz (9) au moment où l'impulsion suivante arrive, dans lequel l'unité de commande est en outre configurée pour commander l'unité laser de manière à combler des espaces restants entre les lésions induites par laser (5a, 5b, 10) dans un passage suivant après l'effondrement des bulles de gaz (9).

5. Dispositif selon l'une quelconque des revendications précédentes,
dans lequel l'unité de commande est configurée pour commander l'unité laser qui conduit à une distribution aléatoire ou quasi aléatoire des lésions induites par laser (5a, 5b, 10) à l'intérieur de la zone d'ouverture.

6. Dispositif selon l'une quelconque des revendications précédentes,
dans lequel l'unité de commande est configurée pour commander l'unité laser pour la génération du diaphragme d'ouverture (1) de telle manière qu'une transmission de lumière à travers la zone d'ouverture du cristallin (2) est réduite à 20 % ou moins.

7. Dispositif selon l'une quelconque des revendications précédentes,
avec l'unité laser pour la génération du diaphragme d'ouverture (1) dans le cristallin (2) de l'œil, dans lequel l'unité laser présente un laser à impulsions pour délivrer des impulsions laser (7, 7b, 7c, 7d), une unité de focalisation pour focaliser les impulsions laser (7, 7b, 7c, 7d) et une unité de redressage pour l'alignement des impulsions laser (7, 7b, 7c, 7d).

8. Dispositif selon la revendication 7,

dans lequel le laser à impulsions est configuré pour délivrer des impulsions laser (7, 7b, 7c, 7d) d'une durée d'impulsion dans la plage de 10 000 à 10 fs, de préférence de 800 à 100 fs, de manière particulièrement préférée de 350 à 150 fs,
avec une énergie d'impulsion dans la plage de 100 à 0,01 $\mu$J, de préférence de 10 à 0,1 $\mu$J, de manière particulièrement préférée de 2 à 0,1 $\mu$J, dans une plage de longueurs d'onde de 400 à 1400 nm, de préférence de 600 à 1 200 nm, de manière particulièrement préférée de 800 à 1100 nm et
avec un taux de répétition dans une plage de 1 à 100 000 kHz, de préférence de 10 à 10 000 kHz, de manière particulièrement préférée de 100 à 500 kHz,
de manière très particulièrement préférée avec une durée d'impulsion de 150 fs, une énergie d'impulsion de 1 $\mu$J, une longueur d'onde dans la plage de 700 à 1100 nm et une fréquence de répétition de 200 kHz.

9. Procédé de génération d'instructions de commande pour une unité laser pour la génération d'un diaphragme d'ouverture (1) dans un œil, dans lequel les instructions de commande amènent l'unité laser à créer le diaphragme d'ouverture (1) dans un cristallin (2) de l'œil, dans lequel le diaphragme d'ouverture (1) sert à augmenter la profondeur de champ de l'œil et est formé par des lésions induites par laser (5a, 5b, 10) qui réduisent une transmission de lumière à travers une zone d'ouverture du cristallin (2) entourant une ouverture,
**caractérisé en ce que** les instructions de commande amènent l'unité laser, en interaction avec une unité d'alignement pour l'alignement et/ou la fixation de l'œil, à créer des repères (12a, 12b) qui définissent l'ouverture dans la direction latérale et/ou axiale (13) du fait du rétrécissement de la pupille par une unité d'excitation de lumière pour l'excitation par la lumière de l'oeil pour entraîner un rétrécissement de la pupille.

10. Programme informatique avec des moyens de programmation permettant à un dispositif selon la revendication 1 d'exécuter un procédé de génération d'un diaphragme d'ouverture (1) dans un œil lorsque le programme informatique est exécuté sur le dispositif.

Fig. 1a

Fig. 1b

Fig. 2

Fig. 3a

Fig. 3b

Fig. 4a    Fig. 4b    Fig. 4c

EP 3 703 629 B1

Fig. 5a　　　Fig. 5b

Fig. 6b

Fig. 6a

Fig. 7a

Fig. 7b

EP 3 703 629 B1

Fig. 7c

Fig. 8a

Fig. 8b

Fig. 8c

Fig. 8d

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2010004641 A1
- US 4955904 A
- US 5757458 A
- US 5980040 A
- WO 2011020078 A1
- US 2013131795 A1
- EP 2231084 B1

**In der Beschreibung aufgeführte Nicht-Patentliteratur**

- **ALIO, JORGE L. et al.** Removability of a small aperture intracorneal inlay for presbyopia correction. *Journal of refractive surgery*, 2013, vol. 29 (8), 550-556